# NOUVEAU FASCICULE DE BREVET EUROPEEN

(11) **EP 3 102 692 B2**
(45) Date de publication et mention de la décision concernant l'opposition: **16.02.2022**
(45) Mention de la délivrance du brevet: 16.01.2019
(21) Numéro de dépôt: 15708563.0
(22) Date de dépôt: 05.02.2015
(51) Int. Cl.: C12Q 1/02, C12Q 1/22, G01N 33/68

(54) **DOSAGE BIOLOGIQUE DES PEPTIDOGLYCANES**
BIOLOGISCHE DOSIERUNG VON PEPTIDOGLYCANEN
BIOLOGICAL DOSAGE OF PEPTIDOGLYCANS

(30) Priorité: 07.02.2014 FR 1450966
(43) Date de publication de la demande: 14.12.2016
(73) Titulaire: Roquette Frères, 62136 Lestrem (FR)
(72) Inventeur: LANOS, Pierre, 59480 La Bassee (FR); BIGUET, Marc, 62840 Neuve Chapelle (FR); BERNARD, Roselyne, 62136 Lestrem (FR); ALLAIN, Fabrice, 59800 Lille (FR); CARPENTIER, Mathieu, 59350 Saint Andre Lez Lille (FR); DENYS, Agnès, 59800 Lille (FR); HACINE-GHERBI, Héla, 59650 Villeneuve D'ascq (FR)
(74) Mandataire: Plasseraud IP
(86) Numéro de dépôt international: PCT/FR2015/050275
(87) Numéro de publication internationale: WO 2015/118267

(56) Documents cités:
- WO-A1-2009/115533
- WO-A1-2012/143647
- WO-A1-2013/178931
- WO-A1-2014/154651
- FR-A1- 2 978 774
- GB-A- 2 481 267
- LI-YUN HUANG ET AL: "Use of toll-like receptor assays to detect and identify microbial contaminants in biological products", JOURNAL OF CLINICAL MICROBIOLOGY, AMERICAN SOCIETY FOR MICROBIOLOGY, UNITED STATES, vol. 47, no. 11, 1 novembre 2009 (2009-11-01), pages 3427-3434, XP002690887, ISSN: 1098-660X, DOI: 10.1128/JCM.00373-09 [extrait le 2009-09-02]
- GRIET GLORIEUX ET AL: "A novel bio-assay increases the detection yield of microbiological impurity of dialysis fluid, in comparison to the LAL-test", NEPHROLOGY DIALYSIS TRANSPLANTATION, OXFORD UNIVERSITY PRESS, GB, vol. 24, no. 2, 1 février 2009 (2009-02-01), pages 548-554, XP002667055, ISSN: 0931-0509, DOI: 10.1093/NDT/GFN485 [extrait le 2008-09-03]
- Goodman et al: J. Bacteriology, vol. 146, 1981, pages 755-763,
- NAKIMBUGWE D et al.: "Cell wall substrate specificity of six different lysozymes and lysozyme inhibitory activity of bacterial extracts", FEMS Microbiol Lett, vol. 259, 21 April 2006 (2006-04-21), pages 41-46,

## Description

### DOMAINE DE L'INVENTION

La présente invention est relative un dosage de peptidoglycanes dans un échantillon, en particulier un échantillon de polymères de glucose.

### CONTEXTE DE L'INVENTION

Les épisodes inflammatoires aseptiques sont des complications majeures observées lors de traitements utilisant des produits manufacturés à des fins thérapeutiques (par exemple : dialyse péritonéale, alimentation parentérale, injection par voie veineuse).

Si une partie de ces épisodes inflammatoires est liée à un problème d'ordre chimique (présence accidentelle de contaminants chimiques ou mauvais dosages de certains composés), la majorité des cas est consécutive à la présence de contaminants d'origine microbienne libérés au cours des processus de fabrication.

Il est maintenant clairement établi que les lipopolysaccharides (LPS) et les peptidoglycanes (PGN) sont les principaux contaminants présentant un risque élevé de déclencher de tels épisodes inflammatoires lorsqu'ils sont présents à l'état de traces dans les produits manufacturés.

Le test LAL (*Limulus Amebocyte Lysate*) est utilisé en routine par de nombreux laboratoires de contrôle-qualité pour détecter et doser les contaminations en LPS. Ce test est basé sur la reconnaissance des endotoxines par un complexe senseur extrait de l'hémolymphe de Limule.

D'autres tests également basés sur la réactivité d'extraits d'hémolymphe d'invertébrés sont actuellement proposés pour la détection des PGN dans les produits à usage thérapeutique (SLP-Wako, Immunetics).

Cependant, ces tests présentent le désavantage de ne pas être très spécifiques, puisqu'ils réagissent également avec d'autres molécules d'origine microbienne, telles que les β-glucanes.

En outre, ces méthodes requièrent l'achat d'appareillage spécifique pour cette utilisation, ce qui augmente grandement les coûts et limite donc l'accès à ces méthodes de dosage.

Par ailleurs, LPS et PGN ont des structures variables en fonction de leur origine bactérienne, ce qui est responsable d'importantes différences de réactivité inflammatoire.

C'est pourquoi il est d'ailleurs nécessaire d'exprimer les résultats des dosages en unités équivalentes de molécules standards (par exemple, LPS *d'E. coli* dans le test LAL).

De plus, ces molécules sont le plus souvent présentes sous forme de complexes macromoléculaires, ce qui affecte leur solubilité et leur potentiel inflammatoire.

Par exemple, les PGN sont très variables en taille et sont souvent agrégés avec d'autres molécules des parois bactériennes, tels que des acides lipoteichoïques et des lipopeptides.

Ainsi, des méthodes "biologiques" ont été développées pour rendre compte uniquement de la charge inflammatoire associée à ces molécules.

Les cellules effectrices de la réponse inflammatoire possèdent des senseurs spécialisés dans la reconnaissance de structures moléculaires spécifiquement produites par les agents infectieux.

Ces molécules, appelée PAMPs pour *pathogen-associated molecular pattern molecules*, sont essentiellement reconnues par les TLRs (acronyme anglo-saxon de *Toll Like Receptors*) et les NLRs (acronyme anglo-saxon de *Nod-like receptors*), dont la spécificité est liée à la structure moléculaire des différentes familles de molécules inflammatoires.

Contrairement aux LPS qui sont des ligands reconnus par les récepteurs de type TLR4, les PGN sont détectés par les récepteurs membranaires de type TLR2, alors que ses produits finaux de dépolymérisation sont reconnus par le récepteur intracellulaire NOD2.

Ces dernières années, des tests cellulaires *in vitro* ont été développés pour remplacer les modèles animaux de réponse inflammatoire.

La plupart de ces tests sont basés sur l'incubation de cellules monocytaires en présence des produits contaminés et sur le dosage en retour de la production en cytokines inflammatoires (TNF-α, IL-1β, IL-6, IL-8, RANTES).

Toutefois, les tests utilisant des cellules primaires isolées du sang sont sujets à une importante variabilité inter-individuelle des donneurs, ce qui peut être responsable de biais expérimentaux.

A l'inverse, les lignées cellulaires monocytaires donnent des réponses constantes, ce qui explique pourquoi elles sont en général préférées aux cellules primaires. Toutefois, ces lignées n'apportent pas non plus entière satisfaction.

Par exemple, le choix des cytokines est souvent critiqué, car la majorité est exprimée de façon transitoire et leur concentration dans le milieu de culture ne reflète pas toujours la charge réelle en molécules inflammatoires.

Etant donné que toutes les cellules monocytaires expriment la majorité des TLRs/NLRs, les tests basés sur leur utilisation ne sont pas sélectifs d'un type de contaminant, mais vont donner une réponse inflammatoire globale.

Par ailleurs, le problème majeur vient des différences de sensibilité des cellules vis-à-vis des différentes molécules inflammatoires.

Ainsi, les PGN, ligands de TLR2, sont nettement moins réactifs que les LPS, ce qui les rend difficilement détectables par ces approches.

En effet, les LPS induisent une réponse significative pour des concentrations de l'ordre du ng/mL, alors que des concentrations 100 fois plus élevées en PGN sont nécessaires pour obtenir une réponse similaire (w/w ratio).

Depuis quelques années, des lignées cellulaires transfectées ont été proposées pour remplacer les modèles précédents dans les tests biologiques de détection et de quantification de la réactivité des composés inflammatoires.

Ces lignées non inflammatoires (par exemple : HEK-293) sont transfectées stablement par un gène codant un récepteur spécifique d'une famille d'agonistes inflammatoires.

Elles contiennent également un vecteur d'expression pour un gène rapporteur codant pour une enzyme (par exemple, la luciférase ou la phosphatase alcaline), dont la synthèse est dépendante de l'activation du récepteur inflammatoire.

Ainsi, la reconnaissance d'un contaminant par les cellules exprimant le bon récepteur va déclencher la synthèse de l'enzyme, dont la production sera suivie par transformation de son substrat en produit coloré ou luminescent.

Ce produit étant facilement quantifiable, cette méthode permet un dosage rapide de la réponse inflammatoire associée à un type de contaminant.

Les avantages de ces modèles cellulaires sont nombreux : remplacement des dosages ELISA des cytokines par un test enzymatique, grande reproductibilité dans les dosages en raison du caractère stable des lignées, ciblage de certaines familles de molécules inflammatoires en fonction du récepteur exprimé, détection des contaminants à des seuils très bas.

Ces modèles cellulaires peuvent donc se substituer aux tests de réponse cytokinique *in vitro,* car ils permettent de cibler spécifiquement les facteurs inflammatoires agonistes d'un TLR ou d'un NLR donné, et de quantifier la réponse inflammatoire associée à cet agoniste.

Par exemple, des cellules exprimant spécifiquement TLR2 et TLR4 ont déjà été utilisées pour la détection de contaminants dans des produits alimentaires (travaux de Clett Erridge du Department of Cardiovascular Sciences de Leicester - UK dans British Journal of Nutrition, vol 105/ issue 01/ Jan 2011, pp 15-23*).*

En outre, des sociétés telles que InvivoGen commercialisent maintenant un large éventail de cellules de la lignée HEK-293 (HEK-Blue^{™}) transfectées avec les différents récepteurs TLRs ou NRLs.

Ces cellules contiennent comme rapporteur un gène codant pour une forme sécrétée de phosphatase alcaline (SEAP: *secreted embryonic alkaline phosphatase*), ce qui permet un dosage colorimétrique aisé et rapide de la réponse aux agonistes inflammatoires.

Ces cellules HEK-Blue^{™} ont déjà été utilisées avec succès pour détecter la présence de contaminants dans des solutions concentrées de polymère de glucose et leur effet synergique (WO2012/143647). WO2013/178931 décrit un procédé permettant de tester l'effet d'étapes de production ou de purification de polymères de glucose sur la présence ou la nature des molécules pro-inflammatoires contaminantes. L'utilisation de lysozyme est mentionnée dans WO2013/178931 et WO2012/143647 pour éliminer les PGN afin de pouvoir doser les autres agonistes TLR2.

Ainsi, il existe toujours un besoin constant de développer des méthodes alternatives et améliorées de dosage de PGN total dans un échantillon, en particulier un échantillon de polymères de glucose.

### RESUME DE L'INVENTION

La présente invention est donc relative à une méthode biologique de dosage des peptidoglycanes dans un échantillon, en particulier un échantillon de polymères de glucose.

En particulier, la présente invention est relative à un procédé de dosage de peptidoglycanes (PGN) dans un échantillon de polymère de glucose, comprenant :
a) le traitement enzymatique de l'échantillon de polymère de glucose par un lysozyme, dans lequel le traitement de l'échantillon comprend l'incubation du lysozyme à une concentration d'environ 250 U/ml dans l'échantillon à une concentration en polymère de glucose de 37,5 % (poids/volume) pendant 2h à une température de 37°C ;
b) la mise en contact de l'échantillon traité ou d'une dilution de celui-ci avec une cellule recombinante exprimant un récepteur TLR2 (Toll-like Receptor 2) exogène et un gène rapporteur sous la dépendance directe de la voie de signalisation associée au récepteur TLR2, le gène rapporteur codant pour une protéine colorée ou fluorescente ou pour une protéine dont l'activité peut être mesurée;
c) la mesure du signal de gène rapporteur ; et
d) la détermination de la quantité de PGN dans l'échantillon à l'aide d'une courbe étalon de correspondance entre la quantité de PGN et l'intensité du signal de gène rapporteur.

De préférence, le traitement enzymatique de l'échantillon permet de fragmenter et désagréger les PGN contenus dans l'échantillon, en particulier de manière à les rendre capable d'activer le récepteur TLR2.

En particulier, le traitement enzymatique de l'échantillon permet de générer des PGN présentant majoritairement une taille d'environ 120 kDa.

De préférence, le traitement enzymatique est réalisé à l'aide d'un lysozyme dont l'activité est capable de désagréger les complexes de PGN dans l'échantillon. En effet, il est question dans la présente invention de libérer des PGN actifs, de tailles comprises entre 30 et 5000 kDa, notamment une taille d'environ 120 kDa.

Des conditions précises de traitement enzymatiques sont choisies à cet effet.

L'enzyme est le lysozyme, utilisée à une concentration d'environ 250 U/ml et mise en présence de l'échantillon à une concentration en polymère de glucose de 37,5 % (poids/volume) pendant 2 h à une température de 37°C.

Ensuite, l'échantillon ainsi traité sera soumis au test avec des cellules recombinantes exprimant le récepteur TLR-2 selon la présente invention.

De préférence, les cellules recombinantes exprimant le récepteur TLR-2 possèdent le gène rapporteur codant pour une phosphatase alcaline sécrétée. Dans un mode de réalisation préféré, la cellule est une cellule de la lignée HEK-Blue^{™} hTLR2.

De préférence, la courbe étalon de correspondance entre la quantité de PGN et l'intensité du signal du gène rapporteur est préparée avec des PGN provenant d'une bactérie choisie parmi *Staphylococcus aureus, Micrococcus luteus*, *Escherichia coli, Bacillus subtilis* et *Alicyclobacillus acidocaldarius*, de préférence *Staphylococcus aureus*, *Micrococcus luteus*, et *Alicyclobacillus acidocaldarius.* Cette courbe étalon peut être standardisée ou calibrée en utilisant un étalon interne agoniste de TLR2, de préférence un lipopeptide, en particulier le PAM₃Cys-Ser-(Lys)4 trihydrochloride.

De manière alternative et préférée, la courbe étalon de correspondance entre la quantité de PGN et l'intensité du signal de gène rapporteur peut être préparée en utilisant un étalon interne agoniste de TLR2, de préférence un lipopeptide, en particulier le PAM3Cys-Ser-(Lys)4 trihydrochloride. En effet, il a été montré par les inventeurs que, de manière tout à fait surprenante, le PAM₃Cys-Ser-(Lys)4 trihydrochloride permet de calibrer la réponse TLR2 aux différents PGN standards. Ainsi, la quantité de PAM₃Cys-Ser-(Lys)4 trihydrochloride en ng/ml correspond à la quantité de PGN « corrigée » en ng/ml. La courbe étalon peut donc être établie avec le PAM₃Cys-Ser-(Lys)4 trihydrochloride de manière tout à fait reproductible, sans avoir besoin d'avoir recours à des PGN standard qui introduisent une variabilité suivant leur origine bactérienne et leur procédé de production.

De manière facultative, le procédé comprend une étape préalable de préparation de la courbe étalon en utilisant un étalon interne agoniste de TLR2, de préférence un lipopeptide, en particulier le PAM₃Cys-Ser-(Lys)4 trihydrochloride.

La description décrit en outre un kit permettant le dosage de peptidoglycanes (PGN) dans un échantillon de polymères de glucose, comprenant :
- une cellule recombinante exprimant un récepteur TLR2 (Toll-like Receptor 2) exogène et un gène rapporteur sous la dépendance directe de la voie de signalisation associée au récepteur TLR2, le gène rapporteur codant pour une protéine colorée ou fluorescente ou pour une protéine dont l'activité peut être mesurée; et
- soit une courbe étalon de correspondance entre la quantité de PGN et l'intensité du signal de gène rapporteur, soit un étalon interne agoniste de TLR2, de préférence un lipopeptide, en particulier le PAM₃Cys-Ser-(Lys)4 trihydrochloride ;
- facultativement une notice d'utilisation et/ou une solution de pré-traitement de l'échantillon, en particulier une enzyme de type lysozyme.

Dans un mode de réalisation particulier, le kit comprend :
- une cellule recombinante exprimant un récepteur TLR2 (Toll-like Receptor 2) exogène et un gène rapporteur sous la dépendance directe de la voie de signalisation associée au récepteur TLR2, le gène rapporteur codant pour une protéine colorée ou fluorescente ou pour une protéine dont l'activité peut être mesurée avec ou sans substrat ; et
- un étalon interne agoniste de TLR2, de préférence un lipopeptide, en particulier le PAM₃Cys-Ser-(Lys)4 trihydrochloride ;
- facultativement une notice d'utilisation et/ou une solution de lysozyme.

### DESCRIPTION DETAILLEE DE L'INVENTION

La présente invention est donc relative à une méthode biologique de dosage des peptidoglycanes dans un échantillon, en particulier un échantillon de polymères de glucose.

En particulier, la présente invention est relative à un procédé de dosage de peptidoglycanes (PGN) dans un échantillon de polymère de glucose, comprenant :
a) le traitement enzymatique de l'échantillon de polymère de glucose par un lysozyme, dans lequel le traitement de l'échantillon comprend l'incubation du lysozyme à une concentration d'environ 250 U/ml dans l'échantillon à une concentration en polymère de glucose de 37,5 % (poids/volume) pendant 2h à une température de 37°C ;
b) la mise en contact de l'échantillon traité ou d'une dilution de celui-ci avec une cellule recombinante exprimant un récepteur TLR2 (Toll-like Receptor 2) exogène et un gène rapporteur sous la dépendance directe de la voie de signalisation associée au récepteur TLR2, le gène rapporteur codant pour une protéine colorée ou fluorescente ou pour une protéine dont l'activité peut être mesurée avec ou sans substrat ;
c) la mesure du signal de gène rapporteur ; et
d) la détermination de la quantité de PGN dans l'échantillon à l'aide d'une courbe étalon de correspondance entre la quantité de PGN et l'intensité du signal de gène rapporteur.

De préférence, les polymères de glucose sont destinés à la dialyse péritonéale, la nutrition entérale et parentérale et l'alimentation des nouveaux nés.

Dans un mode de réalisation préféré, les polymères de glucose qui seront testés sont de l'icodextrine ou des maltodextrines.

En particulier, ils peuvent être destinés à la préparation de dialyse péritonéale. Ils peuvent être testés à un ou plusieurs stades de leur préparation, et notamment au niveau de la matière première, à une étape quelconque de leur procédé de préparation, et/ou au niveau du produit final du procédé. Ils peuvent également être testés en tant qu'échantillon d'une solution de dialyse péritonéale.

Dans une première étape du procédé, l'échantillon de polymère de glucose est traité par voie enzymatique.

Le but de ce traitement est de fragmenter les PGN et/ou de désagréger les PGNs contenus ou piégés dans des agrégats, le but étant de générer des PGNs capables d'interagir avec les récepteurs TLR2 et de les activer.

Comme indiqué précédemment, ce traitement doit permettre de désagréger les PGNs contenus ou piégés dans des agrégats et de fragmenter les PGNs de taille trop élevés, notamment pour générer des PGNs solubles de tailles comprises entre 30 et 5000 kDa, notamment d'environ 120 kDa.

Cependant, le traitement ne doit pas affecter la capacité des PGNs à interagir avec les récepteurs TLR2. Il est de préférence optimisé pour libérer au maximum de PGNs capables d'interagir avec TLR2 et d'activer le récepteur et pour conserver un maximum de PGNs déjà actifs sur TLR2.

Bien entendu, si la concentration en polymère de glucose variait, l'homme du métier devra adapter en conséquence les quantités d'enzyme et/ou la durée d'incubation.

Le lysozyme est utilisé à une concentration de 250 U/mL, pendant 2 h à une température de 37°C

Le lysozyme est une enzyme commercialisée par exemple par la société Euromedex (ref. 5934 ; origine : blanc d'oeuf ; activité ≥ 25000 units/mg). Cependant, il est à noter que l'homme du métier peut également utiliser d'autres lysozymes répondant aux mêmes critères d'activité enzymatique et de degré de pureté.

Dans une étape subséquente, l'échantillon et/ou des dilutions de celui-ci sont mis en contact avec des cellules recombinantes exprimant le récepteur TLR2. Les cellules sont qualifiées de recombinantes car ce sont des cellules qui ont été modifiées par l'introduction d'un acide nucléique codant pour la récepteur TLR2, de préférence le récepteur TLR2 humain, la cellule initiale n'exprimant pas TLR2.

L'activité du récepteur TLR2 est détectée en utilisant un gène rapporteur qui est sous la dépendance directe de la voie de signalisation associée audit récepteur. De manière préférée, ce gène rapporteur code pour une protéine colorée ou fluorescente, ou pour une protéine dont l'activité peut être mesurée avec ou sans substrat.

De manière particulière, le gène rapporteur code pour une phosphatase alcaline. Notamment, le gène rapporteur peut produire une forme sécrétée de la phosphatase alcaline (acronyme anglo-saxon SEAP: *secreted embryonic alkaline phosphatase*), dont la synthèse est sous la dépendance directe de la voie de signalisation associée à TLR2.

Dans un mode de réalisation préférée, la lignée cellulaire utilisée est une lignée HEK-Blue^{™} (commercialisée par la société InvivoGen), modifiées par transfection stable avec des vecteurs codant pour TLR2 humain : la lignée HEK-Blue^{™} hTLR2. Cependant, il est à noter que l'homme du métier peut également utiliser d'autres lignées commercialement (Imgenex) ou il peut en préparer.

Lorsque la cellule est HEK-Blue^{™} hTLR2, la cellule est de préférence mise en oeuvre à une densité d'environ 50 000 cellules/puits pour une plaque de 96 puits.

Ensuite, le procédé comprend la mesure du signal de gène rapporteur.

Dans un mode de réalisation préféré mettant en oeuvre la lignée HEK-Blue^{™} hTLR2, le signal est la mesure de l'activité de la phosphatase alcaline. De préférence, la réaction enzymatique est mise en oeuvre en utilisant un ratio 1 : 3 de milieu à tester versus le réactif SEAP (par exemple 50 µL de milieu et 150 µL de réactif SEAP). En outre, un temps de réaction d'au moins 60 minutes sera préféré.

Enfin, la quantité de PGN dans l'échantillon est déterminée à l'aide d'une courbe étalon de correspondance entre la quantité de PGN et l'intensité du signal de gène rapporteur.

Le standard de PGN est de préférence calibré en utilisant un étalon interne agoniste de TLR2, de façon à exprimer les résultats en unités équivalentes de PGN actif.

L'étalon interne peut être un lipopeptide, de préférence de synthèse, en particulier le PAM₃Cys-Ser-(Lys)4 trihydrochloride (Pam3(cys), PAM ou Pam signifiant acide palmitique).

Ainsi, la courbe étalon de correspondance entre la quantité de PGN et l'intensité du signal de gène rapporteur peut être réalisée directement avec un étalon interne agoniste de TLR2, de préférence un lipopeptide, en particulier le PAM₃Cys-Ser-(Lys)4 trihydrochloride, une quantité de PAM₃Cys-Ser-(Lys)4 trihydrochloride en ng/ml correspondant à la quantité de PGN en ng/ml.

De manière alternative, la courbe étalon de correspondance entre la quantité de PGN et l'intensité du signal de gène rapporteur peut être standardisée ou calibrée avec un étalon interne agoniste de TLR2, de préférence un lipopeptide, en particulier le PAM3Cys-Ser-(Lys)4 trihydrochloride.

Cet étalon interne est de préférence de synthèse ou à une structure/composition bien définie. La calibration ou standardisation est réalisée en comparant les pentes des parties linéaires de chaque courbe dose-réponse et en calculant un facteur de correction permettant de superposer la courbe obtenue avec l'étalon de calibration et celle du standard PGN.

Cette courbe de correspondance entre la quantité de PGN et l'intensité du signal de gène rapporteur peut également être réalisée avec un étalon interne agoniste de TLR2, de préférence un lipopeptide, en particulier le PAM₃Cys-Ser-(Lys)4 trihydrochloride, notamment avec les mêmes cellules, dans les mêmes conditions, avec des doses croissantes d'étalon interne agoniste de TLR2.

De même que pour le PGN, elle peut être réalisée en absence ou en présence de polymère de glucose, de préférence en présence.

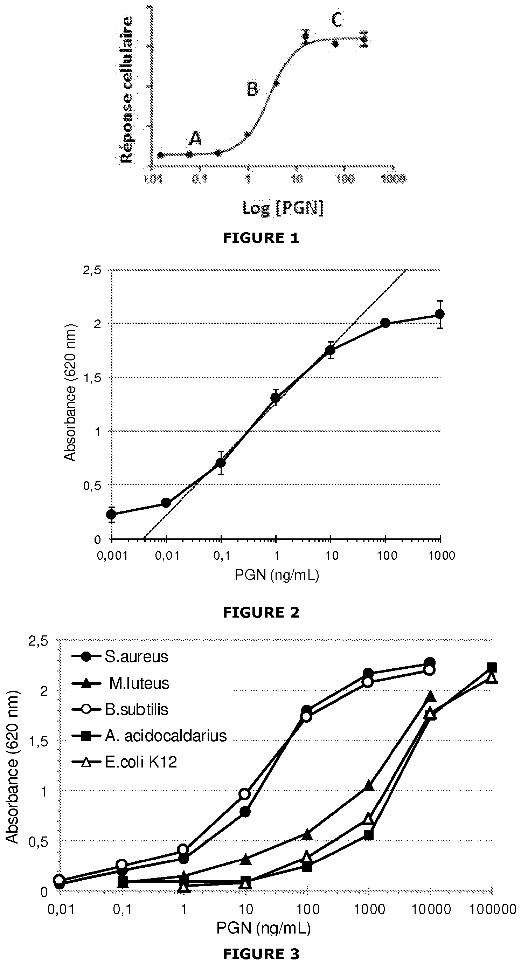

Typiquement, la courbe étalon est une courbe classique de réponse cellulaire de type sigmoïde (Figure 1).
- la partie (A) correspond aux réponses obtenues avec des concentrations faibles en PGN, en-deçà de celles donnant une activation efficace de TLR2. Cette zone non linéaire correspond donc au seuil limite de détection de la méthode. De façon à inclure la variabilité de la méthode, on estime ce seuil de détection à trois fois la valeur du bruit de fond (réponse obtenue en absence de stimulus).
- la partie (B) est la plus intéressante car on observe une réponse linéaire. Cette zone de réponse efficace permet de déterminer une relation directe entre la réponse cellulaire et le taux de PGN. Il s'agit donc de la zone de dosage.
- la partie (C) correspond à une saturation de la réponse cellulaire en présence de concentrations trop fortes en PGN. Il y a en fait une saturation des récepteurs TLR2.

On se place dans la partie linéaire de la courbe étalon, cette partie correspondant à une zone (partie B) où la quantité de PGN est directement proportionnelle au signal du gène rapporteur.

Dans le cas d'échantillons susceptibles d'être fortement contaminés en PGN, il faudra réaliser plusieurs dilutions en série de façon à toujours se localiser dans la zone de linéarité. A l'inverse, des concentrations faibles en PGN nécessitent une étape de concentration de l'échantillon si l'on veut augmenter la sensibilité du dosage.

Facultativement, le procédé comprend en outre un test avec une cellule contrôle qui n'exprime pas TLR2, plus généralement qui n'exprime pas de récepteur de l'immunité innée. Par exemple, la lignée HEK-Blue^{™} Null2 peut être utilisée. Il s'agit d'une lignée contrôle, dont l'utilisation est utile pour vérifier que l'échantillon de polymères de glucose n'induit pas la production de l'enzyme par un mécanisme intrinsèque.

La description décrit également un kit permettant le dosage de peptidoglycanes (PGN) dans un échantillon de polymères de glucose, le kit comprenant :
- une cellule recombinante exprimant un récepteur TLR2 (Toll-like Receptor 2) exogène et un gène rapporteur sous la dépendance directe de la voie de signalisation associée au récepteur TLR2, le gène rapporteur codant pour une protéine colorée ou fluorescente ou pour une protéine dont l'activité peut être mesurée avec ou sans substrat. Notamment, la cellule est de préférence la lignée HEK-Blue^{™} hTLR2. A titre de témoin négatif, le kit peut également comprendre une cellule n'exprimant pas de récepteur de l'immunité innée, par exemple la lignée HEK-Blue^{™} Null2.
- soit une courbe étalon de correspondance entre la quantité de PGN et l'intensité du signal de gène rapporteur, soit un étalon interne agoniste de TLR2, de préférence un lipopeptide, en particulier le PAM₃Cys-Ser-(Lys)4 trihydrochloride. Facultativement, le kit peut comprendre une courbe étalon ainsi qu'un étalon interne agoniste de TLR2, de préférence un lipopeptide, en particulier le PAM₃Cys-Ser-(Lys)4 trihydrochloride. Alternativement, le kit peut comprendre une courbe étalon ainsi qu'un standard calibré de PGN provenant du même microorganisme que celui utilisé pour préparer cette courbe étalon.
- facultativement une notice d'utilisation, une solution de pré-traitement de l'échantillon, les réactifs utiles pour mesurer la réponse du gène rapporteur, des microplaques, etc...Notament, le kit peut comprendre un lysozyme.

### DESCRIPTION DES FIGURES

FIGURE 1 : Courbe théorique de la réponse cellulaire en fonction de concentrations croissantes de PGN.
FIGURE 2 : Courbe étalon de la réponse cellulaire en fonction du taux de PGN de *S*. *aureus* obtenue avec les cellules HEK-Blue^{™}-hTLR2.
FIGURE 3 : Réponse des cellules HEK-Blue^{™}-hTLR2 en fonction des concentrations croissantes en PGN de différentes espèces bactériennes.
FIGURE 4 : Structure du PAM₃Cys-Ser-(Lys)4 trihydrochloride (PAM3(cys)).
FIGURE 5 : Comparaison des réponses induites par différents lots de PGN de S. aureus et par le PAM3(cys) dans les cellules HEK-Blue^{™}-hTLR2.
FIGURE 6 : Réponse des cellules HEK-Blue^{™}-hTLR2 en fonction des concentrations corrigées en PGN.
FIGURE 7 : Courbe étalon de la réponse des cellules HEK-Blue^{™}-hTLR2 en fonction des concentrations corrigées en PGN actif.
FIGURE 8 : Effet de la durée du traitement par le lysozyme sur les réponses cellulaires induites par les échantillons de polymères de glucose.
FIGURE 9 : Comparaison des réponses cellulaires induites par les échantillons de polymères de glucose après traitement par 10 et 100 µg/mL de lysozyme.

### EXEMPLES

Le dosage est basé sur la reconnaissance spécifique des PGNs par une lignée exprimant le récepteur TLR2 et sur la production d'une activité enzymatique mesurable via l'activation de la voie de signalisation associée à TLR2.

### Matériel cellulaire

Pour les expériences relatives à ce dosage, deux lignées sont utilisées :
- lignée HEK-Blue^{™} hTLR2 (HEK-TLR2): réponse spécifique pour les ligands de TLR2, avec une forte réactivité pour les PGN solubles.
- lignée HEK-Blue^{™} Null2 (HEK-Null): réponse non spécifique liée à un effet cytotoxique de l'échantillon.

Les cellules sont cultivées selon les recommandations du fournisseur (InvivoGen). A 75% de confluence, les cellules sont remises en suspension à une densité de 0,28x10⁶ cellules/mL. Avant stimulation, 180 µL de la suspension cellulaire sont répartis dans les puits de culture (boite de 96 puits), soit 50 000 cellules/puits. Les cellules sont ensuite stimulées pendant 24 h par addition de 20 µL des échantillons de polymère de glucose à 37,5 % (poids/volume) (soit une dilution finale des échantillons à 3,75 %). Après 24 h de stimulation, la réponse cellulaire est mesurée par quantification de l'activité enzymatique produite.

### 1- Etablissement de la courbe étalon pour le dosage biologique des PGN avec un étalon interne

Les courbes dose-réponse ont été réalisées en diluant les PGN de différentes espèces bactériennes dans une solution de maltodextrine non contaminée (référencée P-11.11) préparée à 37,5% (poids/volume). Les PGN testés sont extraits de *Staphylococcus aureus* (Sigma, Cat No 77140), *Micrococcus luteus* (Sigma, Cat No 53243), *Bacillus subtilis* (InvivoGen, # tlrl-pgnb2), *Escherichia coli* K12 (InvivoGen, # tlrl-pgnek) et *Alicyclobacillus acidocaldarius* (souche CNCM I-4689).

Les courbes obtenues sont classiques des réponses observées dans les dosages réalisés avec un matériel cellulaire (bio-assay) (Figures 1 et 2). Les valeurs d'absorbance inférieures à 0,2 témoignent de concentrations trop faibles en PGN pour induire une réponse cellulaire, alors que des valeurs supérieures à 2 montrent un effet de plateau lié à la saturation des récepteurs TLR2. Par conséquent, seule la zone comprise entre ces deux valeurs d'absorbances limites permet de corréler la production de SEAP à la quantité de PGN présente dans les échantillons.

Les réponses observées montrent une large variabilité dans la réactivité cellulaire associée à chaque type de PGN. En effet, les concentrations donnant une réponse égale à 50% de la réponse maximale (EC50) sont de ∼ 20 ng/mL pour les PGN de *S*. *aureus* et *B. subtilis*, 1500 ng/mL pour *M*. *luteus,* et plus de 2000 ng/mL pour ceux extraits de *A*. *acidocaldarius* et *E*. *coli* K12 (Figure 3).

Ces différences étaient toutefois attendues, puisque les PGN ont des structures différentes en fonction de leur origine bactérienne, ce qui est responsable d'importantes variations de réactivité inflammatoire. Ces observations soulignent l'importance de définir un standard interne de façon à pouvoir exprimer les résultats en unités équivalentes de PGN.

Un autre facteur susceptible de modifier la réponse des cellules HEK-TLR2 est la taille des PGN, qui va influencer leur solubilité et la réactivité vis-à-vis de TLR2. Ainsi, la procédure de purification de ces macromolécules peut largement influencer la réponse des cellules, puisque les conditions d'extraction vont pouvoir modifier la taille des PGN, voire provoquer une dégradation partielle.

Il apparait donc nécessaire d'introduire un étalon interne pour la courbe étalon, de façon à éviter des erreurs relatives à la variabilité des PGN et à exprimer les résultats en quantité de PGN « actif ».

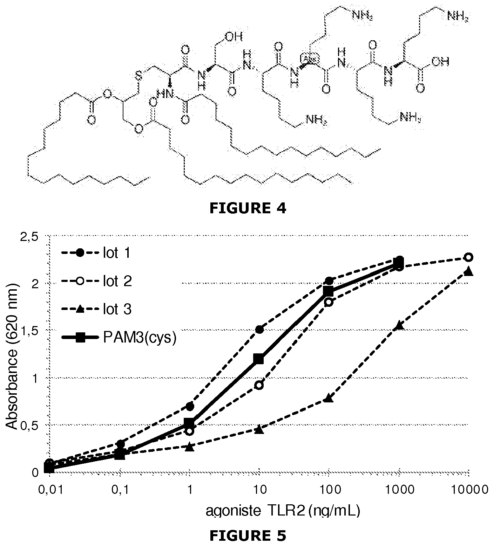

Le PAM₃Cys-Ser-(Lys)4 trihydrochloride (PAM3(cys)) est un lipopeptide de synthèse triacylé qui mime la structure des lipopeptides bactériens et agit comme un agoniste fort de TLR2. Etant de structure homogène, il est souvent utilisé comme témoin positif pour calibrer les réponses des cellules exprimant le récepteur TLR2 (Figure 4).

Pour tester cette hypothèse, les réponses des cellules HEK-TLR2 induites par trois lots distincts de PGN extraits de *S*. *aureus* : 2 lots Sigma (Cat No 77140 : lot 1, 0001442777 ; lot 2, BCBH7886V) et 1 lot InvivoGen (# tlrl-pgnsa), ont été comparées à celles induites par le (PAM₃(cys) (Figure 5).

Les résultats montrent une variabilité de réactivité entre les trois lots de PGN. En effet, les EC50 sont respectivement de 4, 20 et 400 ng/mL pour les trois lots. Ces données indiquent que des PGN extraits de la même espèce bactérienne risquent de présenter des différences de réactivité, même si les lots proviennent du même fournisseur et ont été à priori extraits par la même procédure. Comme attendu, les cellules montrent une forte réactivité vis-à-vis du PAM3(cys), avec une EC50 de 8 ng/mL.

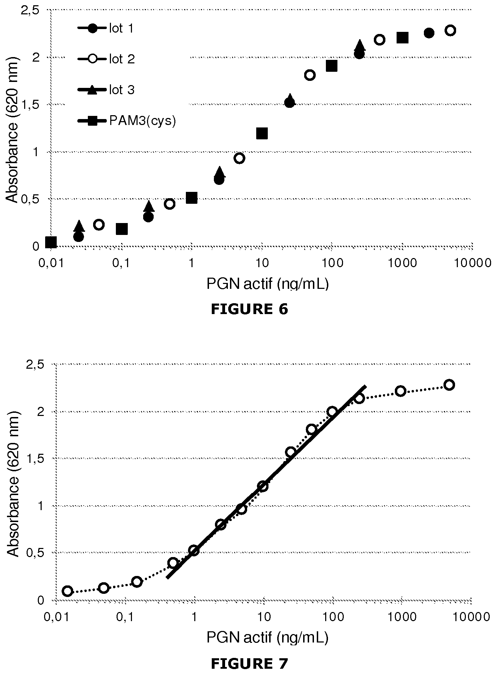

La calibration de chaque lot de PGN par rapport au PAM3(cys) peut être réalisée en comparant les pentes des parties linéaires de chaque courbe dose-réponse, et en calculant un facteur de correction pour superposer les courbes des PGN à celle du PAM3(cys). Dans l'exemple présenté en Figure 6, les facteurs de corrections ont été estimés à 0,4, 2 et 40 respectivement pour les lots 1, 2 et 3. Cela signifie qu'il faut 2,5 fois moins de PGN du lot 1 pour obtenir des réponses identiques à celles induites par le PAM3(cys), mais 2 fois plus de PGN du lot 2 et 40 fois plus de PGN du lot 3. Après avoir corrigé les quantités brutes de PGN, on peut voir que tous les points s'alignent sur une même courbe qui se superpose à celle obtenue avec le PAM3(cys) (Figure 6). L'utilisation de cet étalon interne permet donc d'obtenir des concentrations corrigées pour tous les lots de PGN et d'établir une courbe dose-réponse calibrée en PGN « actif ».

En appliquant cette méthode, la courbe standard de réponse des cellules HEK-TLR2 présente un seuil de détection de 0,07 ng/mL (soit 2 ng/g de polymères de glucose) et une zone de linéarité pour des concentrations de PGN actif comprises entre 0,3 et 200 ng/mL (soit entre 8 et 5400 ng/g de polymères de glucose). (Figure 7)

### 2. Traitement enzymatique des échantillons de polymères de glucose

L'objectif du traitement enzymatique par le lysozyme est de fragmenter et/ou désagréger les PGN contenus dans l'échantillon, de manière à les rendre capables d'induire une réponse inflammatoire via le récepteur TLR2. En particulier, le traitement enzymatique de l'échantillon provoque une dépolymérisation partielle des PGN pour générer des PGN solubles de tailles comprises entre 30 et 5000 kDa, notamment une taille d'environ 120 kDa. Cependant, le traitement enzymatique ne doit pas affecter la capacité des PGN à interagir avec les récepteurs TLR2. Il est de préférence optimisé pour libérer un maximum de PGN solubles capables d'interagir avec TLR2 et pour conserver un maximum de PGN déjà actifs sur TLR2.

Les essais de traitements enzymatiques ont été réalisés sur quatre échantillons standards :
(1) une préparation de maltodextrine non contaminée (référencée P-11.11),
(2) une préparation de maltodextrine P-11.11 artificiellement contaminée par une dose sub-optimale de PGN de *S*. *aureus* (20 ng/mL final) (dénommée P-11.11 + PGN),
(3) une préparation d'icodextrine contaminée (référencée I-209J) et
(4) une matrice de polymère de glucose (référencée E1242).

| | P-11.11 | I-E209J | E1242 |
|---|---|---|---|
| LPS (EU/g) Test LAL | < 0,15 | 0,6 | 2,4 |
| PGN (ng/g) Test SLP-HS | < 3 | 393 | 21 |

Les traitements ont été réalisés sur des solutions de polymères de glucose à 37 % (poids/volume). Puis, les solutions ont été diluées au 1/10^{ème} en présence des cellules, en ajoutant 20 µL de la solution à tester à 180 µL de suspension cellulaire.

Le lysozyme utilisé dans ces expériences est commercialisé par Euromedex (ref. 5934 ; origine : blanc d'oeuf ; activité ≥ 25000 units/mg).

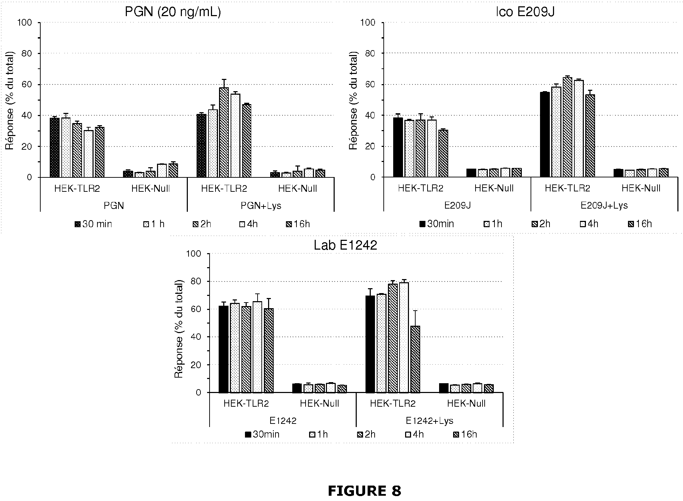

Les premiers essais ont été réalisés en incubant des préparations de maltodextrine P-11.11 contaminée par du PGN (20 ng/mL), d'icodextrine I-209J ou de matrice Iab1920-E1242, en présence de lysozyme à la concentration de 250 U/mL (10 µg/mL). Le traitement a été réalisé à 37°C pendant des temps variant de 30 min à 16 h (Figure 8). Après stimulation des cellules, les résultats montrent que le traitement enzymatique augmente la réactivité des PGN vis-à-vis des cellules HEK-TLR2. De plus, aucune réponse n'est observée dans les cellules HEK-Null, ce qui indique que le traitement par le lysozyme n'a pas d'effet cytotoxique.

L'augmentation de réponse cellulaire induite par le lysozyme est observée pour des temps courts, avec un effet optimal à 2 h de traitement. Par contre, un temps d'hydrolyse plus long réduit la réponse des cellules. Cette observation indique que le traitement prolongé en présence de lysozyme provoque une dépolymérisation trop poussée des PGN, ce qui réduit leur capacité à interagir avec les récepteurs TLR2.

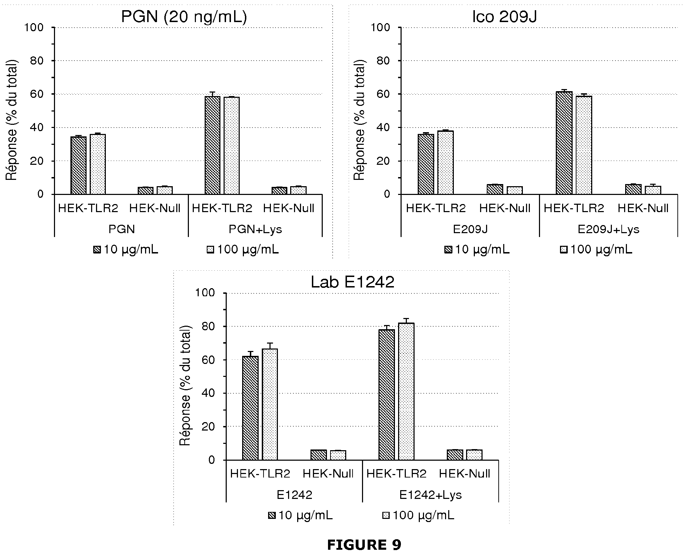

Les expériences ont ensuite été reproduites avec les même échantillons, mais en utilisant le lysozyme aux concentrations 10 et 100 µg/mL (Figure 9). Après 2 h de traitement à 37°C, aucune différence significative dans les réponses cellulaires n'est observée entre les deux concentrations 10 et 100 µg/mL En effet, les traitements enzymatiques augmentent de manière similaire la réactivité des PGN pour les cellules HEK-TLR2, et aucune cytotoxicité n'est observée dans les HEK-Null, même à la plus forte concetrationen lysozyme.

Ces résultats montrent qu'un traitement par le lysozyme à une concentration de 250 U/mL pendant 2 h à 37°C est efficace pour provoquer une dépolymérisation partielle des PGN présents dans des échantillons de polymère de glucose et augmenter leur réactivité vis-à-vis des cellules HEK-TLR2.

En conclusion, dans les conditions optimales définies précédemment, un traitement par le lysozyme est efficace pour augmenter la réactivité des PGN présents dans des préparations de polymères de glucose.

Selon le type du PGN et la nature de l'échantillon de polymère de glucose (produit fini *versus* matrice issue d'une étape de *process*), l'effet activateur provoqué par le traitement enzymatique augmente la réactivité des cellules HEK-TLR2 jusqu'à un facteur 1,5. Ainsi, ce traitement est particulièrement adapté pour convertir toutes les traces de PGN présents dans les échantillons en PGN « actifs » et permettre leur dosage biologique.

## Revendications

1. Procédé de dosage de peptidoglycanes (PGN) dans un échantillon de polymère de glucose, comprenant :
a) le traitement enzymatique de l'échantillon de polymère de glucose par un lysozyme, dans lequel le traitement de l'échantillon comprend l'incubation du lysozyme à une concentration d'environ 250 U/ml dans l'échantillon à une concentration en polymère de glucose de 37,5 % (poids/volume) pendant 2 h à une température de 37°C;
b) la mise en contact de l'échantillon traité ou d'une dilution de celui-ci avec une cellule recombinante exprimant un récepteur TLR2 (Toll-like Receptor 2) exogène et un gène rapporteur sous la dépendance directe de la voie de signalisation associée au récepteur TLR2, le gène rapporteur codant pour une protéine colorée ou fluorescente ou pour une protéine dont l'activité peut être mesurée;
c) la mesure du signal de gène rapporteur ; et
d) la détermination de la quantité de PGN dans l'échantillon à l'aide d'une courbe étalon de correspondance entre la quantité de PGN et l'intensité du signal de gène rapporteur.

2. Procédé selon la revendication 1, dans lequel le traitement enzymatique de l'échantillon permet de fragmenter et désagréger les PGN contenus dans l'échantillon, en particulier de manière à les rendre capable d'activer le récepteur TLR2.

3. Procédé selon l'une ou l'autre des revendications 1 et 2, dans lequel le traitement enzymatique de l'échantillon permet de générer des PGN présentant majoritairement une taille d'environ 120 kDa.

4. Procédé selon l'une quelconque des revendications 1-3, dans lequel le gène rapporteur est une phosphatase alcaline sécrétée.

5. Procédé selon l'une quelconque des revendications 1-4, dans lequel la cellule est une cellule de la lignée HEK-Blue^{™} hTLR2.

6. Procédé selon l'une quelconque des revendications 1-5, dans lequel la courbe étalon de correspondance entre la quantité de PGN et l'intensité du signal de gène rapporteur est établie avec un étalon interne agoniste de TLR2, de préférence un lipopeptide, en particulier le PAM₃Cys-Ser-(Lys)4 trihydrochloride.

7. Procédé selon l'une quelconque des revendications 1-6, dans lequel l'échantillon est dilué, si nécessaire, de manière à générer un signal du gène rapporteur correspondant à la partie linéaire de la courbe étalon.

8. Procédé selon l'une quelconque des revendications 1-7, dans lequel l'échantillon est un échantillon d'une solution d'icodextrine.

## Patentansprüche

1. Verfahren zur Dosierung von Peptidoglycanen (PGN) in einer Glucosepolymer-Probe, umfassend:
a) die enzymatische Behandlung der Glucosepolymer-Probe durch ein Lysozym, wobei die Behandlung der Probe die Inkubation des Lysozyms mit einer Konzentration von ungefähr 250 U/ml in der Probe mit einer Konzentration des Glucosepolymers von 37,5 % (Gewicht/Volumen) während einer Dauer von 2 Stunden bei einer Temperatur von 37 °C umfasst;
b) das Inkontaktbringen der behandelten Probe oder ihrer Lösung mit einer rekombinanten Zelle, welche einen exogenen TLR2-Rezeptor (Toll-like Receptor 2) und ein Reporter-Gen exprimiert, unter dem direkten Einfluss des mit dem TLR2-Rezeptor verbundenen Signalwegs, wobei das Reporter-Gen ein farbiges oder fluoreszierendes Protein kodiert oder ein Protein kodiert, dessen Aktivität gemessen werden kann;
c) Messen des Reporter-Gen-Signals, und
d) Bestimmung der PGN-Menge in der Probe mittels einer Referenzkurve für die Zuordnung der PGN-Menge und der Intensität des Reporter-Gen-Signals.

2. Verfahren nach Anspruch 1, wobei die enzymatische Behandlung der Probe eine Fragmentierung und Zersetzung der in der Probe enthaltenen PGN erlaubt, insbesondere um diesen eine Aktivierung des TLR2-Rezeptors zu ermöglichen.

3. Verfahren nach einem der Ansprüche 1 und 2, wobei es die enzymatische Behandlung der Probe erlaubt, PGN zu erzeugen, die überwiegend eine Größe von ungefähr 120 kDa aufweisen.

4. Verfahren nach einem der Ansprüche 1-3, wobei das Reporter-Gen eine sezernierte alkalische Phosphatase ist.

5. Verfahren nach einem der Ansprüche 1-4, wobei die Zelle eine Zelle der Linie HEK-Blue^{™} hTLR2 ist.

6. Verfahren nach einem der Ansprüche 1-5, wobei die Referenzkurve für die Zuordnung der PGN-Menge und der Intensität des Reporter-Gen-Signals mittels eines internen TLR2-Agonisten-Standards bestimmt wird, vorzugsweise ein Lipopeptid, insbesondere PAM₃Cys-Ser-(Lys)4-Trihydrochlorid.

7. Verfahren nach einem der Ansprüche 1-6, wobei die Probe, falls erforderlich, verdünnt wird, um ein Signal des Reporter-Gens zu erzeugen, das dem linearen Teil der Referenzkurve entspricht.

8. Verfahren nach einem der Ansprüche 1-7, wobei die Probe eine Icodextrin-Probenlösung ist.

## Claims

1. A method of assaying peptidoglycans (PGNs) in a sample of glucose polymer, comprising:
a) enzymatic treatment of the sample of glucose polymer by a lysozyme; wherein the treatment of the sample comprises incubation of the lysozyme at a concentration of approximately 250 U/ml in the sample at a glucose polymer concentration of 37.5% (weight/volume) for 2 h at a temperature of 37°C;
b) bringing the treated sample or a dilution thereof into contact with a recombinant cell expressing an exogenous TLR2 receptor (Toll-like Receptor 2) and a reporter gene under the direct dependence of the signaling pathway associated with the TLR2 receptor, the reporter gene coding for a colored or fluorescent protein or for a protein whose activity can be measured;
c) measuring the reporter gene signal; and
d) determining the amount of PGN in the sample using a calibration curve of the correspondence between the amount of PGN and the intensity of the reporter gene signal.

2. The method as claimed in claim 1, wherein the enzymatic treatment of the sample makes it possible to fragment and disaggregate the PGNs contained in the sample, in particular so as to make them capable of activating the TLR2 receptor.

3. The method as claimed in one or the other of claims 1 and 2, wherein the enzymatic treatment of the sample makes it possible to generate PGNs predominantly having a size of approximately 120 kDa.

4. The method as claimed in any one of claims 1-3, wherein the reporter gene is a secreted alkaline phosphatase.

5. The method as claimed in any one of claims 1-4, wherein the cell is a cell of the HEK-Blue^{™} hTLR2 line.

6. The method as claimed in any one of claims 1-5, wherein the calibration curve of the correspondence between the amount of PGN and the intensity of the reporter gene signal is set up with an internal standard that is an agonist of TLR2, preferably a lipopeptide, in particular PAM₃Cys-Ser-(Lys)4 trihydrochloride.

7. The method as claimed in any one of claims 1-6, wherein the sample is diluted, if necessary, so as to generate a signal of the reporter gene corresponding to the linear portion of the calibration curve.

8. The method as claimed in any one of claims 1-7, wherein the sample is a sample of a solution of icodextrin.
